# NEUE EUROPÄISCHE PATENTSCHRIFT

(11) **EP 1 894 214 B2**
(45) Veröffentlichungstag und Bekanntmachung des Hinweises auf die Entscheidung über den Einspruch: **14.02.2018**
(45) Hinweis auf die Patenterteilung: 19.01.2011
(21) Anmeldenummer: 06762032.8
(22) Anmeldetag: 13.06.2006
(51) Int. Cl.: H01F 1/36, C12N 15/10, H01F 1/11

(54) **MAGNETPARTIKEL MIT EINER GESCHLOSSENEN, ULTRADÜNNEN SILIKASCHICHT, VERFAHREN ZU DEREN HERSTELLUNG UND VERWENDUNG**
MAGNETIC PARTICLES WITH A CLOSED ULTRATHIN SILICA LAYER, METHOD FOR THE PRODUCTION THEREOF AND THEIR USE
PARTICULES MAGNETIQUES COMPORTANT UNE COUCHE DE SILICE FERMEE ULTRAFINE, LEUR PROCEDE DE PRODUCTION, ET LEUR UTILISATION

(30) Priorität: 23.06.2005 EP 05013522
(43) Veröffentlichungstag der Anmeldung: 05.03.2008
(73) Patentinhaber: Siemens Healthcare Diagnostics GmbH, 65760 Eschborn (DE)
(72) Erfinder: HENNIG, Guido, 50859 Köln (DE); HILDENBRAND, Karlheinz, 47802 Krefeld (DE)
(74) Vertreter: Zounek, Nikolai
(86) Internationale Anmeldenummer: PCT/EP2006/005677
(87) Internationale Veröffentlichungsnummer: WO 2006/136314

(56) Entgegenhaltungen:
- WO-A-03/058649
- US-B1- 6 548 264

## Beschreibung

Die vorliegende Erfindung bezieht sich auf magnetische mit Silika (SiO₂) beschichtete Partikel, wobei die Silikatschicht geschlossen und dicht ist und sich durch eine extrem geringe Dicke von maximal 0,5 Nanometern - im Folgenden auch als Silika-Nanoschicht genannt - auszeichnet. Die Erfindung beschreibt ein verbessertes Verfahren zur Herstellung dieser silikathaltigen Magnetpartikel, welches im Vergleich zum Stand der Technik zu einem Produkt mit einer geschlossenen Silikatschicht und dadurch bedingt auch zu einer stark verbesserten Reinheit führt. Darüber hinaus verhindert das neue Verfahren eine unkontrollierte Aggregat- und Clusterbildung von Silikaten an der Magnetitoberfläche, wodurch die im weiteren genannten Eigenschaften und biologischen Anwendungen positiv beeinflusst werden Das neue Verfahren ermöglicht außerdem die Abreicherung von nanopartikulären Feststoffpartikeln auf Basis einer fraktionierten Zentrifugation. Die erfindungsgemäßen Magnetpartikel zeigen ein optimiertes Magnetisierungs- und Suspensionsverhalten sowie ein sehr vorteilhaftes Ablaufverhalten von Kunstoffoberflächen. Diese hochreinen mit Siliziumdioxid beschichteten Magnetpartikel werden bevorzugt für die Isolierung von Nukleinsäuren aus Zell- oder Gewebeproben eingesetzt, wobei die Abtrennung mittels magnetischer Felder aus einer Probenmatrix erfolgt. Diese Partikel eignen sich besonders gut zur automatisierten Aufreinigung von Nukleinsäuren, meistens aus biologischen Körperproben, zwecks deren Nachweis mit verschiedenen Amplifikationsmethoden.

In jüngster Zeit gewinnt die Molekulare Diagnostik zunehmend an Bedeutung. Die Molekulare Diagnostik hat Eingang gefunden in die klinische Diagnostik von Erkrankungen. Dies umfasst die Vermessung von molekularen Markern zur Verbesserung der Diagnosestellung einer Krankheit, der frühzeitigen Erkennung, des Monitorens einer Erkrankung während der Therapie, der Prognose von Erkrankungen sowie der Prädiktion von Wirkungen bzw. Nebenwirkungen von Medikamenten (u.a. Nachweis von Infektionserregern, Nachweis von Mutationen des Genoms, Vorhersage von Wirkung bzw. Nebenwirkungen von Medikamenten anhand von vorgegebenen oder im Laufe einer Erkrankung erworbenen Erbgutmustern, Entdeckung von zirkulierenden Tumorzellen und Identifizierung von Risikofaktoren für die Prädisposition einer Erkrankung). Aber auch in der Veterinärmedizin, der Umweltanalytik und Nahrungsmitteltestung finden Methoden der molekularen Diagnostik mittlerweile ihre Anwendung. Ein weiteres Anwendungsgebiet stellen Untersuchungen an pathologischen/zytologischen Instituten oder im Rahmen forensischer Fragestellungen dar. Aber auch im Rahmen der Gesundheitsvorsorge (z.B. Untersuchungen von Blutkonserven auf Infektionserreger-Freiheit) wird die Gen-Diagnostik mittlerweile eingesetzt und der Gesetzgeber plant, solche Testungen per Gesetz in Zukunft anzuordnen. Methoden, die auch bei der klinischen Molekularen Diagnostik zum Einsatz kommen (wie z.B. Hybridisierungs- oder Amplifikationstechniken wie die PCR (Polymerase Chain Reaction), TMA (Transcription Mediated Amplification), LCR (Ligase Chain Reaction), bDNA (branched DNA) oder NASBA-(Nucleic Acid Sequence Based Amplification) gehören auch bei wissenschaftlichen Grundlagenarbeiten zu den Routineverfahren.

Voraussetzung für die Durchführung eines Assays in der Molekularen Diagnostik ist im Allgemeinen die Isolierung von DNA oder RNA aus der zu analysierenden Probe. Zwar gibt es Analyseverfahren, wie z.B. bDNA-basierte Tests, die es erlauben, Nukleinsäureisolierung und Nachweisreaktion gemeinsam durchzuführen, doch erfordert die PCR als die in der Molekularen Diagnostik am weitesten verbreitete molekularbiologische Methode wegen ihrer Beeinflussbarkeit durch exogene Faktoren nahezu immer die Verwendung von vorher gereinigten Nukleinsäuren.

Die klassischen Präparationsverfahren für Nukleinsäuren beruhen dabei auf einer Flüssig-Flüssig Extraktion. Als Beispiel sei hier die Phenol-Chloroform Extraktion von DNA aus Körperproben genannt. Der hohe Arbeitsaufwand und die Notwendigkeit, teilweise hochtoxische Substanzen verwenden zu müssen, hat diese Verfahren jedoch in den vergangenen Jahren stark zu Gunsten von festphasebasierten Verfahren in den Hintergrund treten lassen.

Bei der Verwendung von festphasebasierten Extraktionsverfahren von Nukleinsäuren lässt sich die Probenvorbereitung zum eigentlichen Analysengang, weitgehend unabhängig von der jeweiligen Fragestellung in vier Grundschritte unterteilen: 1. Konditionierung der festen Phase; 2. selektive oder spezifische Bindung des Analyten an die feste Phase und Entfernung der übrigen Probematrix; 3. Herauswaschen von eventuellen Verunreinigungen aus der festen Phase und 4. Elution des angereicherten und gereinigten Analyten.

Hinsichtlich selektiver und reversibler Bindung von Nukleinsäuren macht man sich die lange bekannte Eigenschaft von Nukleinsäuren zu nutze, unter chaotropen oder anderen Hochsalzbedingungen, d.h. bei hohen Konzentrationen von chaotropen oder anderen Salzen, spezifisch an silikathaltige Adsorbentien wie Glasmehl [Proc. Natl. Acad. USA 76 (1979) 615-619 , Anal. Biochem. 121 (1982) 382-387], diamatomeenerde [Methods Enzymol. 65 (1979) 176-182] oder native Kieselerde [J. Clin. Microbiol. 28 (1990) 495-503 , EP 0 389 063 B1] zu binden. Mit Hilfe eines ein wasserlösliches organisches Lösungsmittel enthaltenen Puffers, in der Regel ist das ein niederer aliphatischer Alkohol, werden dann Verunreinigungen von dem Adsorbens gewaschen, der Träger getrocknet und die adsorbierten Nukleinsäuren mit destilliertem Wasser oder einem sogenannten Niedrigsalzpuffer, d. h. Puffer mit geringer Ionenstärke, eluiert.

Im Hinblick auf vollständige und kostengünstige Automatisierung der Nukleinsäureisolierung spielen Verfahren mit superparamagnetischen Adsorbentien eine zunehmend wichtige Rolle.

Im einfachsten Ausführungsfall (WO 01/46404) werden kommerziell hergestellte Magnetpartikel, die für technische Anwendungen, wie bspw. elektrographische Toner hergestellt werden, ohne weitere Modifizierung direkt zur Nukleinsäureaufbereitung eingesetzt.

Derartige großtechnisch hergestellte Produkte erfüllen zwar einige der wichtigsten Voraussetzungen, wie eine gewisse Nukleinsäureadsorption und Magnetisierbarkeit. Auf der anderen Seite können diese kommerziell verfügbaren Produkte wichtige Randbedingungen, die hinsichtlich hochsensitiver und reproduzierbarer Ergebnisse unabdingbar sind, nicht erfüllen. Zum Beispiel ist es gerade auf dem Gebiet der Virusdiagnostik (z.B. HCV oder HIV) von entscheidender Bedeutung die viralen Nukleinsäuren quantitativ aus dem Serum oder Plasma - also mit nahezu 100% Ausbeute - zu extrahieren um daraus eine akkurate Viruskonzentration im Serum/Plasma abzuleiten und damit Therapieentscheidungen zu treffen. Vor dem Hintergrund der optischen Auswertung spielt auch die Reinheit der Magnetpartikel eine entscheidende Rolle. Gerade bei Magnetitpartikeln, die häufig noch mikroporös sind, kann es durch Diffusion von Eisenatomen aus dem Partikel heraus zu gefärbten Lösungen kommen, die Transmissions- oder Reflektionsmessungen empfindlich stören können.

Es sind deshalb zahlreiche Entwicklungen von Magnetpartikeln für biologische Anwendungen, speziell im Hinblick auf manuelle und automatisierte Nukleinsäureisolierung, beschrieben.

Hierbei spielen magnetische Partikel, die an der Oberfläche eine hohe Dichte von SiOH Gruppen tragen, eine überragende Rolle. Von SiOH Gruppen ist nämlich bekannt, dass sie reversible Bindungen mit Nukleinsäuren eingehen können.

Im Hinblick auf hochsensitive, quantitative und reproduzierbare Ergebnisse müssen derartige Magnetpartikel neben Magnetisierbarkeit und Nukleinsäurebindungsvermögen eine Reihe weiterer Randbedingungen erfüllen, die im Folgenden näher beschrieben werden.

### Teilchengröße und Teilchengrößenverteilung:

Es hat sich gezeigt, dass magnetische Partikel aus Fe₃O₄(Magnetit), die bspw. von der Fa. Lanxess unter dem Namen Bayoxide E für elektrographische Toner mit Primärpartikelgrößen im Bereich von ca. 0.1 bis 1 µm verfügbar sind, bzgl. Partikelgröße nahezu ideale Voraussetzungen erfüllen. Mit derartigen Partikelgrößen kann nämlich die wichtige Randbedingung der für biologische Anwendungen wichtigen "Suspensionsstabilität" erreicht werden. Diese muss einerseits so beständig sein, dass es nach Schütteln innerhalb einiger Minuten, bspw. 10 bis 15 Minuten (Adsorptionsphase der Nukleinsäuren) zu keiner nennenswerten Sedimentation kommt, während nach Anlegung des Magnetfeldes die mit Nukleinsäuren beladenen Magnetpartikel im Hinblick auf möglichst kurze Analysezeiten innerhalb weniger Minuten, bspw. innerhalb 1 bis 5 Minuten, vollständig abgetrennt werden müssen.

In diesem Zusammenhang weisen die verfügbaren Magnetpartikel aus Fe₃O₄(Magnetit) leider noch dahingehend Mängel auf, dass auch geringe Mengen an sehr feinen Magnetitpartikeln im Nanometerbereich enthalten sind.

Diese unerwünschten Nebenprodukte, die wegen ihrer großen Oberfläche beträchtliche Mengen Nukleinsäuren binden können, werden im Magnetfeld leider nicht innerhalb weniger Minuten abgetrennt und somit kann der Informationsgehalt dieser Nukleinsäuren - gerade im Hinblick auf quantitative Vermessung der Nukleinsäuren - verloren gehen.

Neben diesen Ausbeuteverlusten führt das auch zu nicht klaren, oft gelbbraunen Überständen, die sowohl die kommerzielle Vermarktung negativ beeinflussen können als auch bei der photometrischen Auswertung der Eluate stören.

Es wäre daher von großem Vorteil wenn dieser "nanopartikuläre Magnetitpartikelanteil" für die hier beschriebene biologischen Anwendung abgetrennt werden könnte.

### Silikatgehalt:

Wie oben erwähnt, besitzen einige großtechnisch hergestellte Magnetpartikel, bspw. die Bayoxide E Serie der Fa. Lanxess auch ohne spezielle Silika Nachbehandlung ein gewisses Nukleinsäurebindungsvermögen, da sie herstellbedingt im "bulk" und damit in geringem Maße auch an der Oberfläche SiOH Gruppen tragen. Wegen der geringen Nucleinsäureadsorptionskapazität werden bei solchen Produkten entsprechend relativ große Magnetpartikelmengen benötigt, wodurch die Aufarbeitung kleiner Probenvolumina erschwert wird.

Außerdem besitzen derartige Produkte ein für die hier beschriebene Anwendung ungünstiges Benetzungsverhalten von Gefäßwänden, wie Glas oder den Kunststoffwänden in Mikrotiterplatten, die bei der Nukleinsäureaufreinigung routinemäßig eingesetzt werden. So bleiben in wässerigen Suspensionen erhebliche Mengen der nicht modifizierten, relativ hydrophoben Magnetitpartikel an den Mikrotiterplattenwänden adsorbiert und führen so zu Pipettierungenauigkeiten und Ausbeuteverlusten.

Sehr günstig verhalten sich in diesem Zusammenhang Partikel mit einer hohen Dichte von SiOH Oberflächengruppen, die wegen ihrer Hydrophilie sehr vorteilhaft insbesondere von Kunststoffwänden wie z.B. den genannten Mikrotiterplatten abperlen.

Bei vielen Magnetpartikelentwicklungen für die Nucleinsäureisolierung ist dementsprechend der Silikaanteil gegenüber dem Magnetitanteil dominant. Wie bspw. in WO 01/71732 beschrieben, erhält man durch Hydrolyse von reaktiven Silikaverbindungen, wie bspw. Tetraethoxysilan (TEOS) in der Anwesenheit von Magnetitpartikeln, Silikapartikel, die durch den Magnetiteinschluss magnetisierbar sind. Wegen der hohen Dichte an SiOH Gruppen an der Oberfläche zeigen derartige Partikel zwar eine hohe Nukleinsäurebindungskapazität sowie ein günstiges Benetzungsverhalten der Mikrotiterplattenwände, auf der anderen Seite sind jedoch, dem verringerten Magnetitgehalt entsprechend, die magnetischen Eigenschaften stark reduziert. Außerdem besitzen die so hergestellten magnetischen Silikapartikel, deutlich ungünstigere morphologische Eigenschaften, wie z.B. sehr heterogene Teilchengröße und Teilchengrößenverteilung, wobei zu erwähnen ist, dass große, nicht kugelförmige Teilchen zur Verstopfung bei den automatischen Pipettiervorgängen führen können.

### Extrahierbare Bestandteile:

Die nach dem Magnetpartikelverfahren isolierten Nukleinsäuren werden in der Regel weiteren Prozessen, wie bspw. einer PCR (Polymerase Chain Reaction), TMA (Transcription Mediated Amplification), LCR (Ligase Chain Reaction oder NASBA-(Nucleic Acid Sequence Based Amplification) unterworfen. Es handelt sich hierbei um hochsensible, enzymgesteuerte Verfahren, die durch zahlreiche Verunreinigungen und Eisenverbindungen, die bspw. als Enzymgifte wirken können, gestört werden.

Daher müssen die für die Nukleinsäureaufreinigung hergestellten Magnetpartikel besondere Reinheitsanforderungen erfüllen. Werden großtechnisch hergestellte Eisenoxide, wie bspw. Bayoxide der Fa. Lanxess eingesetzt, so ist diese Aufgabenstellung durchaus nicht trivial, da die Magnetitpartikel eine gewisse Porosität sowie eine Oberflächenrauhigkeit aufweisen. Daher können sowohl aus dem Prozess der Eisenoxidherstellung als auch bei der nachfolgenden Silikabehandlung Verunreinigungen in die Mikroporen eingeschlossen werden, die bei späteren Prozessen als Enzymgifte oder im Falle von gefärbten Verunreinigungen bei der photometrischen Auswertung stören können.

### Aufgabe der vorliegenden Erfindung:

Ausgehend von kommerziell verfügbaren Magnetpartikeln sollen Silika modifizierte Magnetpartikel mit einer hohen Dichte an SiOH Oberflächengruppen und einer geschlossenen und dichten Oberflächenschicht aus Silikat hergestellt werden. Durch die Silikamodifizierung sollen weder die Morphologie noch die sehr guten magnetischen Eigenschaften der Ausgangsprodukte wesentlich beeinflusst werden. Ebenso soll das Benetzungsverhalten an Kunststoffoberflächen durch die Silikabeschichtung positiv beeinflusst werden. Darüber hinaus sollen die Silika modifizierten Magnetpartikel hinsichtlich extrahierbarer Verunreinigungen so weit optimiert sein, dass ein Austreten von Verunreinigungen bzw. Eisenverbindungen aus dem Magnetitkern verhindert wird und weder eine Störung der biologischen Nachweisreaktionen noch der photometrischen Auswertung möglich sind.

### Nächstliegender Stand der Technik:

Ausgehend von Magnetpartikeln Bayoxide E der Fa. Lanxess beschreibt WO 03/058649 ein elegantes Verfahren zur Silika Abscheidung an der Partikeloberfläche mit Wasserglaslösungen, bspw. Wasserglas HK 30 der Fa. Cognis. Durch ein schrittweises Verdünnen des pH Wertes in der Bayoxide E / Wasserglaslösung, gleichbedeutend mit einer schrittweisen pH Verschiebung vom stark alkalischen (pH 11.5) zum Neutralen (pH 7) kommt es zu einer schonenden Abscheidung von Silika an der Magnetpartikeloberfläche Erfolgt, wie in WO 03/058649 erwähnt, die pH Absenkung durch Zugabe von Säuren (WO 98/31840), so kann es an der Säureeintropfstelle zur unkontrollierten Umwandlung von Wasserglas zu Silika (SiO₂) kommen in deren Struktur Magnetpartikel eingelagert werden, sodass die oben erwähnte kontrollierte Silika Abscheidung an der Magnetpartikeloberfläche bei weitem nicht erreicht wird.. Trotzdem läst sich in dem in WO 03/058649 beschriebenen "Batchverfahren" die Bildung von kleinsten Silikaaggregaten bzw-Clustern an der Oberfäche nicht vollständig verhindern.

Während also die in WO 03/058649 beschriebenen Silika modifizierten Magnetpartikel bzgl. Oberflächenstruktur und Nukleinsäurebindungsverhalten gute Eigenschaften zeigten, konnte im Langzeitverhalten (nach einigen Wochen Standzeit) der entsprechenden wässerigen Suspensionen sehr nachteilige gelbbraune Überstände beobachtet werden. In biologischen Assay's bei denen in der Regel auch Tenside eingesetzt werden, ist dieser Effekt bereits nach kürzeren Standzeiten zu beobachten. Analytisch konnten in diesen gefärbten Überständen neben Wasserglaskomponenten, Spuren von Eisenverbindungen sowie sehr feine Magnetitpartikel nachgewiesen werden. Offensichtlich waren diese Verunreinigungen durch die Silikaoberfläche in die poröse Magnetpartikelstruktur eingeschlossen, von wo sie im Laufe der Zeit nach außen diffundierten. Diese Beobachtungen deuten zudem darauf hin, dass die Silikatschicht mit dem in WO 03/058649 beschriebenen Batchverfahren nicht vollständig geschlossen bzw. unregelmäßig verteilt ist und somit das Austreten von Eisenverbindungen nicht verhindern kann.

Philipse, A. P. Et al., Magnetic Silica Dispersions: Preparation and Stability of Surface-Modified Silica Particles with a Magnetic Core, Langmuir 1994, 10, 92-99 beschreiben Partikel, welche eine Silikaschichtdicke von ca. 0,5 nm aufweisen. Diese Partikel werden jedoch nicht durch Absenken des pH auf neutrale Werte erhalten, sondern durch Absenken auf einen pH von 10. Es wird davon abgeraten auf neutrale pH Werte abzusenken.

### Detaillierte Beschreibung der vorliegenden Erfindung

Im Hinblick auf Eliminierung der eben genannten extrahierbaren Störkomponenten, wurden die im Folgenden beschriebenen verfahrenstechnischen Optimierungen durchgeführt, wobei der Fortschritt durch Vergleich mit dem in WO 03/058649 beschriebenen Verfahren dokumentiert wird. Allerdings wurde bei den hier beschriebenen Versuchen im Gegensatz zu den Beispielen von WO 03/058649 nicht Bayoxide E 8707, das nicht mehr als Standardprodukt verfügbar ist, sondern die sehr ähnliche Type Bayoxide E 8706 eingesetzt. In beiden Fällen handelt es sich um Fe₃O₄ Magnetite, die herstellbedingt einen geringen Anteil Si enthalten, wobei bei der Type 8707 der Fe/Si Gehalt 99.1/0.9 und bei Bayoxide E 8706 99.4/0.4 beträgt. Wichtig im Hinblick auf das erfindungsgemäße Verfahren ist die Oberflächenbeschaffenheit, v.a. der pH Wert der Fe₃O₄Magnetite. Während Bayoxide E 8707 mit pH 6.5 eine leicht acide Oberfläche besitzt, wurde bei der nun eingesetzten Type Bayoxide E 8706 ein neutraler pH Wert, je nach Charge sogar leicht alkalische Werte (pH 7.5) ermittelt. Überraschenderweise wurde gefunden, dass selbst diese leicht alkalischen Oberflächeneigenschaften die Wasserglas - Silika Abscheidung induzieren können. Üblicherweise erfolgt die Silikaabscheidung aus den stark alkalischen Wasserglaslösungen durch Zugabe von Säuren.

In den Vergleichsversuchen wurde nun überraschender Weise gefunden, dass bzgl. extrahierbarer Bestandteile deutlich bessere Ergebnisse erzielt werden können, wenn anstelle des in WO 03/058649 beschriebenen schrittweisen pH Absenkens ein kontinuierliches Verfahren, wie bspw. ein Membranverfahren eingesetzt wird. Hierbei wurde, wie in den Beispielen näher beschrieben, die wässrige Wasserglas/Magnetpartikel Suspension nach einer Reaktionszeit von einer Stunde mittels "cross flow Mikrofiltration" aufgereinigt. Bei der "cross flow Mikrofiltration", die bei geringem Überdruck durchgeführt wird, handelt es sich, wie in "Basic Principles of Membrane Technology" von M. Mulder beschrieben, um eine bekannte Trenn- bzw. Aufreinigungsmethode. Es wird hierbei bei konstanten Volumina gearbeitet, d.h. der die Verunreinigungen enthaltende Permeatvolumenstrom wird durch den gleichen Volumenstrom an zulaufendem Frischwasser ersetzt. Im Gegensatz zu dem in der Biologie bekannten Dialyseverfahren können bei der Mikrofiltration dem Porendurchmesser entsprechend nicht nur niedermolekulare Salze sondern auch partikuläre Verunreinigungen abgetrennt werden. Dieser kontinuierliche Reinigungsprozess wurde solange durchgeführt, bis die abfließende Permeatqualität dem Reinheitsgrad des zulaufenden Frischwassers entsprach, was je nach Ansatzgröße ca. 12 bis 15 Stunden dauerte.

Bei der oberflächenanalytischen Charakterisierung mittels ESCA wurde nun völlig überraschend festgestellt, dass die so hergestellten Silika modifizierten Magnetpartikel im Hinblick auf die Silika Oberfläche eine neuartige, nämlich ultradünne Silikastruktur aufweisen, mit der ggf. die verbesserte Aufreinigung bzw. die erhöhte Reinheit korreliert werden kann. Diese Silika-Nanoschicht zeichnet sich durch eine gleichmäßig über die ganze Partikeloberfläche verteilte Silikatschicht von bis zu 0.5 nm aus. Darüber hinaus beschreibt das erfindungsgemäße Verfahren aber auch Schichtdicken von 0.5 nm bis 0.2 nm. Die so beschichteten Partikel haben eine Oberflächenbeschichtung, die dadurch charakterisiert ist, dass sie z.B. den Austritt von Eisenionen in die umgebende Lösung verhindert.

Die Herstellung eines Magnetpartikels mit einer Silikaschichtdicke von 0.2 nm ist in Bsp. 3 beschrieben.

Des weiteren zeichnet sich das erfindungsgemäße Verfahren durch eine geschlossene und dichte Silikatschicht aus, was auch mit der verbesserten Reinheit bzw. verringerten beobachteten Dreckeffekten im Überstand korreliert. Die Reinheit der nach diesem erfindungsgemäßen Verfahren hergestellten silikabeschichteten Magnetpartikel ist im Vergleich zu dem in WO 03/058649 beschriebenen Verfahren wesentlich besser. Somit treten sichtbare Verfärbungen des Überstandes nach der Herstellung und Waschung nicht mehr auf (s. Beispiel 2 und 3). Insbesondere verhindert die dichte und geschlossene Silikaschicht das Austreten von sichtbar oder auch unsichtbaren Verunreinigungen, bspw. Eisenionen, die die Amplifikationsmethoden bzw. die optische Auswertung biologischer Experimente stören können. (s. Beispiel 4 und 5).

Darüber hinaus wurde überraschenderweise gefunden, dass die Bildung von Aggregat- und Clusterbildung von Silikaten an der Magnetitoberfläche durch die langsame und kontinuierliche Verdünnung und somit Absenkung des pH Wertes auf neutrale Werte in dem beschriebenen Membranfiltrationsverfahren nahezu vollständig verhindert bzw. im Vergleich zum in WO 03/058649 beschriebenen "Batchverfahren" noch mal stark reduziert wird. Diese wohldefinierte Nanoschicht von Silizium beeinflusst die im folgenden beschriebene Eigenschaften und biologischen Anwendungen positiv.

Des weiteren wurde gefunden, dass zusätzliche Produktoptimierungen hinsichtlich klarer Überstände dadurch erzielt werden können, dass im Anschluss an das Membranverfahren eine fraktionierte Zentrifugation, die eine Abtrennung langsam sedimentierender Eisenoxidpartikel ermöglicht, durchgeführt wird.

Bei den so hergestellten Proben, die als wässerige Suspensionen gehandhabt werden, konnten alle Kriterien, wie eine dem Ausgangsprodukt absolut identische Magnetisierbarkeit, unveränderte Morphologie, hohe Nukleinsäurebindungskapazität, günstiges Abperlen von den Mikrotiterplattenwänden, hervorragende Suspensionsstabilität bei problemloser Abtrennung der Magnetpartikel im Magnetfeld innerhalb weniger Minuten ohne nennenswerte Verunreinigungen im Überstand, erreicht werden.

Der Ausdruck "magnetische mit Silika beschichtete Partikel" umfasst Magnetit-Kerne, die mit einer Nanoschicht aus Silika beschichtet sind.

Der Ausdruck "geschlossene und dichte Silikaschicht" umfasst eine gleichmäßige, homogene, ein bis mehrfache molekulare Silikaschicht im Bereich einer Schichtdicke von 0,5 bis 0.2 nm. Diese geschlossene Silikaschicht verhindert insbesondere den Austritt von Eisenverbindungen und Eisenionen in die Umgebung des silikabeschichteten Magnetpartikels.

Der Ausdruck "verbessertes Verfahren zur Herstellung" umfasst einen leicht durchführbaren aber sehr intensiven Waschprozess mit Hilfe einer Mikro- bzw. Ultrafiltrationseinheit, der zu einer extremen Reinheit des silikabeschichteten Magnetpartikels führt. In diesem Verfahren kommt es nach einer initialen Präzipitation der Nanoschicht von Silikat auf der Partikeloberfläche zu einer langsamen, kontrollierten und kontinuierlichen Verdünnung und damit Absenkung des pH Wertes auf neutrale pH-Werte in der Reaktionslösung wodurch eine extrem gleichmäßige, dichte, geschlossene und homogene Silikatschicht sich auf der Magnetitoberfläche ausbildet. Des Weiteren werden störende Aggregat- oder Clusterbildungen von Silikaten verhindert bzw. weitgehend reduziert.

Der Ausdruck "Abreicherung von nanopartikulären Bestandteilen mit Hilfe der Zentrifugationstechnik" umfasst die Anwendung von Zentrifugations- oder einfachen Gravitationstechniken. Dabei kommt es zur Sedimentation der gewünschten Fraktionen, während die unerwünschten nanopartikulären Bestandteile durch Abnahme des Überstandes verworfen werden können. Durch Bestimmung der Teilchengrößenverteilung mittels Ultrazentrifugation kann dieser Effekt anhand der abgereicherten kleinteiligen Fraktionen nachgewiesen werden. Bei der Zentrifugnationstechnik wird die Ausgangssuspension 15 min zentrifugiert bei ca. 3000g, der Überstand abgenommen und eine gleiche Menge Wasser oder Puffer hinzu gegeben, resuspendiert und diese Schritte mehrmals (bis zu 10x wiederholt. Bei der Gravitationstechnik wird anstelle der Zenrtrifugation einfach eine längere Zeit gewartet bis sich ein Grossteil der Partikel am Boden des Gefäßes abgesetzt hat und anschließend der wässrige Überstand ausgetauscht.

Der Ausdruck "optimales Magnetisierungsverhalten" umfasst die Eigenschaft der erfindungsgemäßen Partikel einen möglichst großen Magnetitanteil zu haben und somit unter Anlegen eines Magnetfeldes von außen an ein Reaktionsgefäß innerhalb weniger Minuten, bspw. innerhalb 1 bis 5 Minuten, vollständig von der Probenmatrix während der Aufreinigung abgetrennt zu werden. Dies ist insbesondere im Hinblick auf möglichst kurze Aufreinigungszeiten in einem automatisierten Verfahren auf einem Pipettierroboter und die Verwendung von möglichst kostengünstigen Magneten mit begrenzten Magnetfeldstärken als Hardwarekomponenete zu berücksichtigen.

Der Ausdruck "Suspensionsverhalten" umfasst die Eigenschaft der erfindungsgemäßen Partikel aufgrund einer optimale Korngrößenverteilung sich so zu verhalten, dass es während der Aufreinigungsphase nach Schütteln innerhalb einiger Minuten, bspw. 10 bis 15 Minuten (Adsorptionsphase der Nukleinsäuren) zu keiner nennenswerten Sedimentation kommt.

Der Ausdruck "optimales Ablaufverhalten von Kunststoffoberflächen" umfasst die Eigenschaft der erfindungsgemäßen Partikel aufgrund einer hydrophilen Oberflächenbeschaffenheit eine geringe Affinität zu den in biologischen Aufreinigungsprozessen verwendeten Kunststoffartikeln zu besitzen. Die verwendeten Kunststoffartikel umfassen u.a. Polystyrol, Polyethylen- und Polypropylengefäße bzw. sogenannte Mikrotiterplatten aus vergleichbaren Kunststoffen jeglicher Form und Größe. Die spezifische Silikaschicht der erfindungsgemäßen Magnetpartikel ermöglicht dabei eine abstoßende Wechselwirkung mit diesen Kunststoffoberflächen, sodass die beschichteten Magnetpartikel von diesen Oberflächen abperlen und keine größeren Wechselwirkungen eingehen, welche letztendlich in einem biologischen Aufreinigungsverfahren von Nukleinsäuren zu einem Ausbeuteverlust führen können.

Der Ausdruck "Isolierung" umfasst die Aufreinigung der Nukleinsäure aus einer biologischen Probe unter Verwendung der oben genannten silikabeschichteten Magnetpartikel und teilt sich im wesentlichen in folgende Einzelschritte auf:
a) Aufschließen der Probe in einem Reaktionsgefäß mit einem Lysepuffer und nach Inkubation Zugabe von einem Bindungspuffer, welcher vorzugsweise sogenannte chaotrope Salze, besonders bevorzugt Guanidin(ium)isothiocyanat, in hoher Molarität enthält
b) Zugabe von silikatbeschichteten Magnetpartikeln
c) Inkubation bei einer Temperatur, bei der die Nukleinsäure an die Magnetpartikel bindet
d) Entfernung nicht gebundener Bestandteile aus dem Reaktionsansatz durch Anlegen eines Magnetfeldes, welches die Magnetpartikel von der umliegenden Flüssigkeit abtrennt
e) Mehrmaliges Zugeben eines Waschpuffers mit anschließender Entnahme desgleichen bei Magnetisierung der Partikel zur Reinigung der Nukleinsäure von unspezifisch gebundenen Molekülen
f) Zugabe eines Elutionspuffers unter Bedingungen, wo die Nukleinsäure von dem Magnetpartikel getrennt wird
g) Abtrennen des Eluats mit der Nukleinsäure nach erneutem Anlegen eines Magnetfeldes

Der Ausdruck "automatisierte Aufreinigung" umfasst Varianten dieses Verfahren, in denen die manuelle Arbeitskraft von menschlichem Personal ganz oder auch nur in Teilschritten ersetzt und insbesondere bei den Schritten der Aufschließung der biologischen Körperprobe mit einem speziellen Puffer, der Zugabe von magnetischen Partikeln, der Inkubation bei einer bestimmten Temperatur, der Entfernung nicht absorbierter Probenbestandteile, den Waschschritten, der Elution gebundener Nukleinsäuren von den Partikeln bei einer bestimmten Temperatur und dem Abtrennen des Eluats von der Partikelsuspension Anwendung findet.

Der Ausdruck "Nukleinsäuren" umfasst oligomere und polymere Ribonukleotide bzw. 2'-Desoxyribonukleotide mit einer Kettenlänge von mehr als 10 Monomereinheiten. Die Monomereinheiten in Nukleinsäuren sind über Phosphorsäurediesterbindungen zwischen 3'- und 5'-Hydroxylgruppe benachbarter Monomereinheiten verknüpft und an das 1'-Atom der jeweiligen Kohlenhydratkomponente ist glykosidisch eine heterocyclische Base gebunden. Nukleinsäuren können durch Ausbildung von intermolekularen Wasserstoffbrückenbindungen Doppel- und Dreifachstränge ausbilden.

Ebenso sind Protein-/Nukleinsäurekomplexe sowie Nukleinsäuren mit synthetischen Nukleotiden, wie Morpholinos oder PNA's (peptide nucleic acids), gemeint.

Der Ausdruck "biologische Körperprobe" umfasst nukleinsäurehaltiges, biologisches Material, wie z.B. Gesamtblut, Blutserum oder Blutplasma, insbesondere virushaltiges Serum oder Plasma, dabei ganz besonders HIV und HCV infizierte Serumproben, "Buffy Coat" (= weiße Blutkörperchenfraktion des Blutes), Faeces, Ascites, Abstriche, Sputum, Organpunktate, Biopsien, Gewebeschnitte, dabei ganz besonders unterschiedlich fixierte, insbesondere mit formalinhaltigen Fixantien, und in paraffin-eingebettete Gewebeschnitte, Sekrete, Liquor, Galle, Lymphflüssigkeit, Urin, Stuhl, Sperma, Zellen und Zellkulturen. Ebenso kann es sich um Nukleinsäuren handeln, welche aus biochemischen Prozessen stammen und anschließend aufgereinigt werden sollen.

Der Ausdruck "Nachweis mit verschiedenen Amplifikationsmethoden" umfasst die Vervielfältigung der aufgereinigten Nukleinsäuren mit Hilfe verschiedener molekularbiologischer Technologien, insbesondere der PCR, der transkriptionsmediierten Amplifikation (TMA), LCA oder auch NASBA und die anschließende oder simultane Detektion der Amplifikationsprodukte. Ebenso ist der Nachweis mit Signalamplifikationsmethoden, wie z.B. der bDNA, also ohne Nukleinsäureamplifikation gemeint. Die Detektion insbesondere der PCR kann durch die Anwendung kinetischer Verfahren mit Hilfe der Fluoreszenztechnologie auch unter "real-time" Bedingungen durchgeführt werden oder auf einem klassischen Agarose-Gel erfolgen. Insbesondere die "real-time" PCR ermöglicht eine sehr gute quantitative Bestimmung von Nukleinsäuren unter Verwendung von entsprechenden Kalibratoren. Kritisch und limitierend für die klinische Sensitivität (Vermeidung von falsch negativen Ergebnissen) ist dabei die effiziente Aufreinigung der Nukleinsäuren, d.h. effiziente Bindung an das Magnetpartikel und reversible Freisetzung unter PCR kompatiblen Bedingungen.

Mit der vorliegenden Erfindung ist es möglich, aufgrund der Verwendung von speziell hergestellten silikabeschichteten Magnetpartikeln besonders effizient, automatisiert und quantitativ Nukleinsäuren aus biologischen Körperproben aufzureinigen und mit entsprechenden Amplifikationstechniken nachzuweisen.

Somit stellt die vorliegende Erfindung einen wichtigen Beitrag für die Nukleinsäurediagnostik dar.

### Beispiele

Nachfolgend werden beispielhaft Protokolle zur Durchführung der beschriebenen Erfindung gegeben. In diesen Beispielen werden exakte Reaktionsbedingungen für die jeweilige aufzureinigende Nukleinsäure angegeben, allerdings können verschiedene Parameter, wie z.B. Magnetpartikelmenge, Inkubations- und Waschtemperatur, Inkubations- und Waschzeiten sowie die Konzentration von Lysispuffer, Waschpuffer und Elutionspuffer, in Abhängigkeit von der jeweiligen auzureinigenden Nukleinsäure variiert werden.

### Beispiel 1: (Master) Herstellen von silikatbeschichteten Magnetit-Partikeln aus Bayoxide E 8706 mit Wasserglas 37/40 durch schrittweise Absenkung des pH Wertes (analog dem Verfahren aus WO 03/058649 A1)

### Reaktionsteil:

In einem 61 Dreihalskolben mit KPG Rührer werden 4000 g Wasserglaslösung 37/40 (Cognis GmbH) vorgelegt. Unter Rühren werden 2000 g Bayoxide 8706 (Bayer AG) innerhalb von 10 Minuten zugegeben. Anschließend wird noch eine Stunde bei RT nachgerührt.

### Aufarbeitung:

Nach Abschalten des Rührers setzen sich die Silka beschichteten Magnetitbeads ab. Dieser Prozess kann durch Anlegen eines Magnetfeldes ggf. beschleunigt werden. Nach einer Wartezeit von einer Stunde wird der Überstand abgesaugt. Zur Aufarbeitung werden 41 Wasser zugegeben wobei ca. 10 Min gerührt wird. Der Überstand wird wiederum abgesaugt. Dieser Waschprozess wird noch mindestens viermal wiederholt, bis das letzte Waschwasser einen pH Wert von 7.5-7.0 erreicht hat.

**Eigenschaften der Silika-Magnetpartikel:**

| | |
|---|---|
| **Zeta Potenzial:** | - 50.2 |
| **Silika Gehalt nach ESCA:** | 7.0 Atom % Si |

**Reinheit:** Nach 10 tägigem Stehen bei RT war der Überstand gelb/braun gefärbt.

### Beispiel 2: Herstellen von hochreinen silikatbeschichteten Magnetit-Partikeln aus Bayoxide E 8706 mit Wasserglas 37/40, kontinuierliche Absenkung des pH Wertes durch "cross flow Mikrofiltration"

Der in Beispiel 1 beschriebene Reaktionsteil wurde wiederholt, die Aufarbeitung erfolgte jedoch nicht schritt- bzw. batchweise, sondern mit Hilfe der Mikrofiltrationseinheit "Centramate®" der Fa. PALL mit einer 0.2 µm Supor® Membrankassette.

Dazu wurde die Magnetpartikelsuspension mit Hilfe einer Pumpe über einen Schlauch abgesaugt und über die Membrankassette geleitet, wobei das Permeat verworfen wurde, während das Retentat in das Reaktionsgefäß zurückgeleitet wurde. Die dem Permeat equivalente Menge wurde der Partikelsuspension neu zugeführt.

Nach einer Filtrationszeit von 12 h hatte das Permeat bzgl. pH und Leitfähigkeit die Qualität des Orginalwassers erreicht und der Reinigungsprozess war beendet.

**Eigenschaften des Endproduktes:**

| | |
|---|---|
| **Zeta Potenzial:** | - 41 mV |
| **Si Gehalt:** | 4.9 Atom% Si bestimmt nach ESCA. Silika Gehalt des Ausgangsproduktes Bayoxide 8706: 2.4 Atom % Si. |

Der Differenzbetrag 2.5 Atom % Si wurde dementsprechend durch die Silikabehandlung mit Wasserglas an der Partikeloberfläche abgeschieden. Daraus errechnet sich eine Silikaschichtdicke von 0.4 nm.

**Reinheit:** Die durch Ultrafiltration aufgereinigte Partikelsuspension zeigte auch nach mehrmonatigem Stehen bei RT keine Verfärbung im Überstand.

### Beispiel 3:

### Herstellen von hochreinen silikatbeschichteten Magnetit-Partikeln aus Bayoxide E 8706 und Wasserglas 37/40 unter kontinuierlicher Absenkung des pH Wertes durch "cross flow Mikrofiltration" und anschließende fraktionierte Zentrifugation

Das in Bsp. 2 beschriebene Endprodukt wurde mit Hilfe einer Zentrifuge (Eppendorf 5810) bei 3225 g 7 Minuten lang zentrifugiert. Während der Hauptteil (> 98%) des Produktes sedimentiert war, verblieb ein dunkelbraun gefärbter Überstand der verworfen wurde.

Der Rückstand wurde wiederum in Wasser aufgenommen, zentrifugiert und vom farbigen Überstand abgetrennt. Diese fraktionierte Zentrifugation wurde noch 8 mal wiederholt, bis der Überstand farblos war.

**Eigenschaften des Endproduktes (Neuntes Zentrifugat):**

| | |
|---|---|
| **Zeta Potenzial:** | - 35 mV |
| **Si Gehalt:** | 3.0% Si. Der Differenzbetrag 0.6 Atom % wurde dementsprechend durch die Silikabehandlung mit Wasserglas an der Partikeloberfläche abgeschieden. Daraus errechnet sich eine Silikaschichtdicke von 0.2 nm. |

**Reinheit:** Der Überstand der so hergestellten Magnetpartikelsuspension blieb auch bei mehrmonatigem Lagern völlig farblos.

Diese Produktqualität zeigte insbesondere hinsichtlich magnetische Abtrennung hervorragende Werte. So konnte nach Anlegen eines Magneten bereits nach weniger als 20 Sekunden ein absolut klarer Überstand beobachtet werden.

### Beispiel 4: Optische Vermessung von wässrigen Überständen aus silikabeschichteten Magnetpartikelsuspensionen

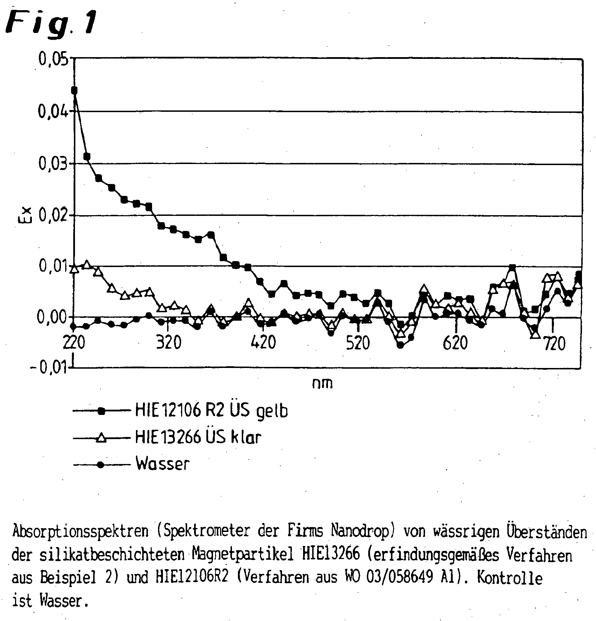

In diesem Experiment wurden die Absorptionspsektren von zwei wässrigen Überständen der silikabeschichteten Magnetpartikel mit der Lotbezeichnung HIE13266 (entstammt dem erfindungsgemäßen Verfahren aus Beispiel 2) und 3) und der Lotbezeichnung HIE12106R2 (entstammt aus dem Verfahren aus WO 03/058649 A1, basierend auf dem Bayoxide E 8707) mit dem Spektrometer der Firma Nanodrop in einem Bereich von 221 - 750 nm aufgenommen (s. Abbildung 1).

Als Referenz wurde ein Wasserspektrum aufgenommen, welches von diesen Spektren abgezogen wurde. Als Kontrolle wurde noch mal ein Wasserspektrum als Probe aufgenommen (Nulllinie).

Aus den Spektren wird ersichtlich, dass die wässrigen Überstände der silikabeschichteten Magnetpartikel HIE13266 ein ähnliches Absorptionsverhalten wie Wasser haben. Hingegen zeigen die Absorptionslinien der Überstände von HIE12106R2 ein deutlich verändertes und erhöhtes Absorptionsverhalten bis in einen Bereich von ca. 500 nm.

Daraus wird ersichtlich, dass das neue, erfindungsgemäße Herstellverfahren mit kontinuierlichem Waschen (Partikel HIE13266) in einer Mikrofiltrationseinheit im Vergleich zu dem Partikel HIE12106R2 mit sequentiellem mehrmaligem Waschen bzw. schrittweisen absenken des pH-Wertes (s.a. WO 03/058649 A1) zu verringerten Dreckeffekten bzw. Austreten von Eisenverbindungen im Überstand führt. Diese Dreckeffekte bei Partikel HIE12106R2 äußern sich durch sichtliche Verfärbung der Überstände über die Zeit sowie das erhöhte Absorptionsverhalten. Darüber hinaus indizieren diese verringerten Dreckeffekte im Überstand eine geschlossene Silikaschicht auf den Partikeln aus dem erfindungsgemäßen Verfahren.

### Beispiel 5: Verhalten von wässrigen Überständen der silikabeschichteten Magnetpartikeln bei der RT-PCR

Es wurden die wässrigen Überstände der zwei unterschiedlich silikabeschichteten Magnetpartikeln (Lotbezeichnungen HIE13266 und HIE12106R2) unter Magnetisierung abgenommen. Partikellot HIE13266 wurde mit dem erfindungsgemäßen Herstellverfahren mit kontinuierlichem Waschen in einer Mikrofiltrationseinheit hergestellt (s. Beispiel 2 und 3). Partikellot HIE12106R2 wurde durch sequentielles mehrmaliges Waschen hergestellt (s. WO 03/058649 A1, basierend auf dem Bayoxide E 8707). Beide Überstände wurden anschließend zu einer quantitativen Einschritt RT-PCR hinzugespikt:
Die so genannte quantitative Einschritt RT (Reverse Transkription)-PCR wurde auf dem MX4000 von Stratagene durchgeführt. Dabei wurden 5 µl der Überstände der einzelnen beiden Partikelüberstände bzw. 5 µl Wasser als Kontrolle zu 20 µl Mastermix gegeben. Dieser beinhaltet folgende Komponenten: 400 nM Primer A, 400 nM Primer B, 10 ng MCF-7 RNA (Ambion), Taqman Primer 200 nM, 1x Buffer A, 5 mM MgCl 2; 1,2 mM dNTPs, 8 U RNaseInhibitor, 20 U MuLV Reverse Transcriptase, 1.25 U Taq Gold (alles von Applied Biosystems). Das PCR Programm lautete: 30 min at 45°C, 10 min bei 95°C, 45 Zyklen mit 15 sec bei 96°C, 60 sec bei 63°C und 30 sec bei 72°C).
Die Ansätze wurden in eine 96well Mikrotiterplatte (Stratagene) gegeben und nach Verschließen ins Analysegerät gestellt. Nach dem Lauf wird mit Hilfe der Gerätesoftware bei einem gewählten Basiswert (Fluoreszenzintensität) im exponentiellen Amplifikationsbereich der Signalkurven jeder Probe ein individueller C t-Wert (Zykluszahl, wo der gewählte Basiswert die Amplifikationskurve schneidet) zugeordnet.

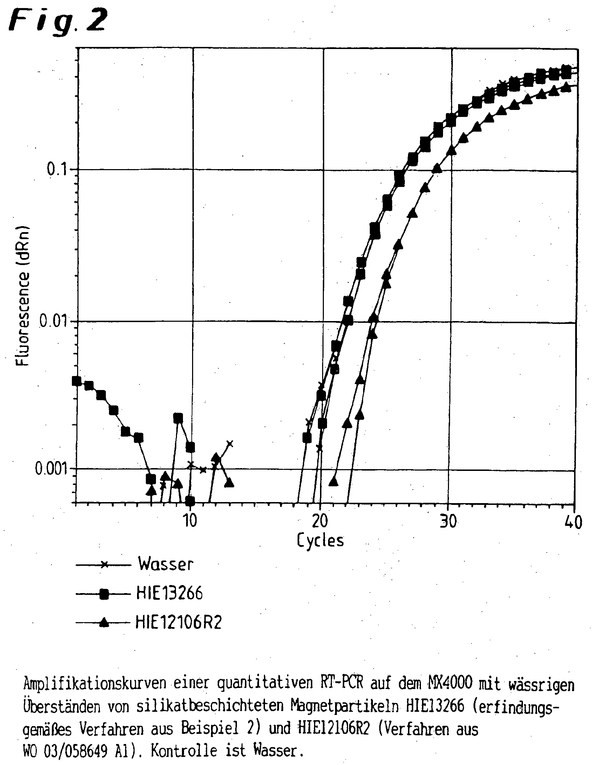

Aus der Abbildung 2 wird ersichtlich das die Amplifikationskurven mit Überständen von Partikel HIE13266 vergleichbar zu den Amplifikationskurven mit Wasser als Probe sind. Hingegen sieht man eine rechtsseitige Verschiebung der Amplifikationskurven um etwa 3 Ct-Werte mit Überständen aus HIE12106R2, was auf eine Interferenz bzw. negative Beeinflussung der RT-PCR Effizienz hindeutet.

## Patentansprüche

1. Verfahren zur Herstellung von silikabeschichteten Magnetpartikeln, welche eine geschlossene Oberflächenbeschichtung mit Silikat mit einer maximalen Schichtdicke von 0,5 nm haben, wobei die initiale Silikatabscheidung aus Wasserglas oder Kieselsol auf den Magnetitpartikeln durch die Oberflächeneigenschaften des Eisenpartikels ausgelöst wird, **dadurch gekennzeichnet, dass** anschließend die Oberfläche durch langsame, kontinuierliche Verdünnung und Absenkung des pH Wertes auf neutrale pH Werte geglättet und abgedichtet wird.

2. Verfahren nach Anspruch 1, wobei das magnetische Material Eisenoxid bzw. Magnetit ist.

3. Verfahren nach Anspruch 2 **dadurch gekennzeichnet, dass** die Korngrößenverteilung zwischen 0,1 und 1 µm liegt

4. Verfahren zur Aufreinigung von Nukleinsäuren aus biologischen Körperproben unter Verwendung von silikabeschichteten Magnetpartikeln, welche eine geschlossene Oberflächenbeschichtung mit Silikat mit einer maximalen Schichtdicke von 0,5 nm haben.

5. Verfahren nach Anspruch 4, wobei das magnetische Material Eisenoxid bzw. Magnetit ist.

6. Verfahren nach Anspruch 4 **dadurch gekennzeichnet, dass** die Korngrößenverteilung zwischen 0,1 und 1 µm liegt.

7. Verfahren nach Anspruch 4 **dadurch gekennzeichnet, dass** es sich bei der Nukleinsäure um RNA oder DNA handelt

8. Verfahren nach Anspruch 4, **dadurch gekennzeichnet, dass** es sich RNA von HCV oder HIV handelt.

9. Verfahren nach Anspruch 4, **dadurch gekennzeichnet, dass** es sich RNA oder DNA aus fixierten Körperproben handelt.

## Claims

1. Method for the production of silica-coated magnetic particles, which have a closed surface coating of silica having a maximum layer thickness of 0.5 nm, wherein the initial silicate deposition from water glass or silica deposit on the magnetic particles is triggered by the surface properties of the iron particles, **characterized in that** the surface is then smoothed and sealed by slow, continuous dilution and reduction of the pH to neutral pH values.

2. Method according to Claim 1, wherein the magnetic material is iron oxide or magnetite.

3. Method according to Claim 2, **characterized in that** the grain size distribution is between 0.1 and 1 µm.

4. Method for the purification of nucleic acids from biological body samples using silica-coated magnetic particles which have a closed surface coating of silica having a maximum layer thickness of 0.5 nm.

5. Method according to Claim 4, wherein the magnetic material is iron oxide or magnetite.

6. Method according to Claim 4, **characterized in that** the grain size distribution is between 0.1 and 1 µm.

7. Method according to Claim 4, **characterized in that** the nucleic acid is RNA or DNA.

8. Method according to Claim 4, **characterized in that** the RNA is from HCV or HIV.

9. Method according to Claim 4, **characterized in that** the RNA or DNA is from fixed body samples.

## Revendications

1. Procédé de production de particules magnétiques revêtues de silice, lesquelles ont un revêtement superficiel fermé de silicate d'une épaisseur de couche maximum de 0,5 nm, dans lequel le dépôt initial de silicate en verre liquide ou sol de silice sur les particules de magnétite est déclenché par les propriétés de surface de la particule de fer, **caractérisé en ce qu'**ensuite la surface est lissée et densifiée par une dilution continue et lente et un abaissement de la valeur du pH à des valeurs de pH neutres.

2. Procédé selon la revendication 1, dans lequel le matériau magnétique est de l'oxyde de fer ou de la magnétite.

3. Procédé selon la revendication 2, **caractérisé en ce que** la distribution granulométrique est entre 0,1 et 1 µm.

4. Procédé de purification d'acides nucléiques d'échantillons corporels biologiques en utilisant des particules magnétiques revêtues de silice, lesquelles ont un revêtement superficiel fermé de silicate d'une épaisseur de couche maximum de 0,5 nm.

5. Procédé selon la revendication 4, dans lequel le matériau magnétique est de l'oxyde de fer ou de la magnétite.

6. Procédé selon la revendication 4, **caractérisé en ce que** la distribution granulométrique est entre 0,1 et 1 µm.

7. Procédé selon la revendication 4, **caractérisé en ce que** l'acide nucléique est de l'ARN ou de l'ADN.

8. Procédé selon la revendication 4, **caractérisé en ce qu'**il s'agit d'ARN de VHC ou de VIH.

9. Procédé selon la revendication 4, **caractérisé en ce qu'**il s'agit d'ARN ou d'ADN d'échantillons corporels fixés.
